# EUROPEAN PATENT APPLICATION

(11) **EP 2 028 132 A1**
(43) Date of publication of application: **25.02.2009**
(21) Application number: 08252761.5
(22) Date of filing: 20.08.2008
(51) Int. Cl.: B65D 83/14, B65D 83/16

(54) **Improvements in or relating to dispensing apparatus**

(30) Priority: 21.08.2007 GB 0716327
(71) Applicant: Consort Medical plc (Formerly Bespak plc), King's Lynn Norfolk PE30 2JJ (GB)
(72) Inventor: Warby, Richard, Cambridgeshire PE13 8AF (GB); Allsop, Paul, Norfolk PE30 3XD (GB)
(74) Representative: Thomson, Neil David

(57) **Abstract**

A dispensing apparatus comprising a dispensing container (8) assembled with a metering valve (1). The dispensing container (8) defines a storage volume for a product and the dispensing container (8) comprises a neck (15) terminating in an open top for receiving the metering valve (1). The open top extends radially outwardly relative to the neck to define a support surface (17).

The metering valve (1) comprises a valve assembly and a ferrule (6), wherein the valve assembly comprises:
a metering chamber body (2) surrounding a metering chamber volume;
an inner seal (3), for sealing an inlet of the metering chamber volume;
an outer seal (4), for sealing an outlet of the metering chamber volume;
a valve stem (5); and
a biasing means (7) for biasing the valve stem (5).

The valve stem (5) is movable between a dispensing position, in which the outlet of the metering chamber volume is open and the inlet of the metering chamber volume is closed, and a rest position, in which the outlet of the metering chamber volume is closed and the inlet of the metering chamber volume is open, wherein the inner seal (3) is supported by the support surface (17) of the open top of the dispensing container (8) when the metering valve (1) is assembled with the dispensing container (8).

## Description

This invention relates to a dispensing apparatus and a metering valve for use with such a dispensing container. In particular, the invention relates to pressurised dispensing apparatus and metering valves.

Prior art metering valves typically include a metering chamber body having an inner seal for sealing an inlet to the metering chamber body and an outer seal for sealing an outlet from the metering chamber body. The valves also include what is known as a valve body which, either, surrounds or supports the metering chamber body and houses one or more parts of the valve assembly, including, the inner and outer seals, the metering chamber, the valve stem, the spring, and the like. In particular, the role of the valve body is to provide a rigid backbone to the valve which helps to hold the valve together. The valve body often provides an abutment for the spring of the valve which is used to bias the valve stem into a rest position. Further, often either a ferrule or a container interacts directly with the valve body to establish connection of the valve to the container.

There is a need in the pharmaceutical field for lower-metered dose and/or lower-volume pressurised dispensing containers for specific drug applications, which necessitates providing both containers and valves in smaller sizes. Further, in order to reduce material costs, reduce complexity of assembly and to reduce the overall size of the valves, there is a need for a valve and a dispensing apparatus having a fewer number of total parts. However, the requirement for a valve body as part of prior art metering valves leads to difficulties in reducing the overall size of the valve.

According to the present invention there is provided a dispensing apparatus comprising a dispensing container assembled with a metering valve;
the dispensing container defining a storage volume for a product, the dispensing container comprising a neck terminating in an open top for receiving the metering valve; the open top extending radially outwardly relative to the neck to define a support surface;
the metering valve comprising a valve assembly and a ferrule wherein the valve assembly comprises:
a metering chamber body surrounding a metering chamber volume;
an inner seal, for sealing an inlet of the metering chamber volume;
an outer seal, for sealing an outlet of the metering chamber volume;
a valve stem; and
a biasing means for biasing the valve stem;
the valve stem being movable between a dispensing position, in which the outlet of the metering chamber volume is open and the inlet of the metering chamber volume is closed, and a rest position, in which the outlet of the metering chamber volume is closed and the inlet of the metering chamber volume is open;
wherein the inner seal is supported by the support surface of the open top of the dispensing container when the metering valve is assembled with the dispensing container.

Advantageously, the metering valve of the dispensing apparatus does not include a valve body. Instead the inner seal and metering chamber body are directly supported by the dispensing container. This allows the overall height of the metering valve to be reduced. In deed, with the exception of the inner end of the valve stem, the inner seal is the innermost component of the metering valve when in the dispensing position. The metering valve has a reduced number of components compared to prior art valves and is as a result easier to manufacture in small sizes and assemble.

Preferably direct contact between the inner seal and the dispensing container acts as a gasket seal between the dispensing container and the metering valve.

Advantageously with the present valve no separate gasket seal is required between the dispensing container and the metering valve since the inner seal, by virtue of its location and the absence of a valve body, is able to act as both the sliding inner seal between the metering chamber body and the valve stem and as a gasket seal preventing leakage of product from the dispensing container to atmosphere at the junction between the metering valve and the dispensing container. As a result the total volume of seal material required in the valve is lower than in many prior art valves. This has an advantage because the degree to which material components of the seal material may leach into the stored product during the lifetime of the apparatus is partly dependant on the volume of the seal material present. Thus it is an advantage to have a reduced volume of seal material.

Preferably the support surface is formed by a portion of the open top of the dispensing container located between the neck and a distal rim of the open top.

Preferably the support surface is orientated substantially perpendicular to a longitudinal axis of the valve stem. This orientation provides the most straightforward means for sealing against a substantially planar, annular inner seal. However the support surface could be angled in a different manner, for example the support surface could be a conical face angled at approximately 45 degrees to the longitudinal axis of the valve stem.

Preferably the support surface is annular. It is preferred that the support surface contacts the inner seal around the entire annulus of the dispensing apparatus in order to provide the most efficient support to the inner seal and to help prevent localised distortion of the inner seal on movement of the valve stem. In addition the contact around the entire annulus is important where the inner seal is also acting as a gasket seal for the dispensing apparatus. Additional sealing features such as one or more raised beads or rings may be provided on the sealing surface to increase the sealing effect.

Preferably the open top defines a recess for locating the metering valve in engagement with the dispensing container. Also preferably the recess is bounded by an upturned rim of the open top of the dispensing container. This geometry facilitates assembly since the recess allows easy location of the metering valve prior to crimping of a ferrule over the valve and onto the dispensing container. The upturned rim in which the metering valve is received also prevents lateral movement of the metering valve relative to the dispensing container during use.

Preferably the support surface is formed by a portion of the open top of the dispensing container located between the neck and the upturned rim of the open top.

Preferably the dispensing container further comprises a flask portion, wherein the minimum diameter of the neck is smaller than the maximum diameter of the flask portion. In this way the flask portion can be sized and shaped to meet requirements such as the volume of product to be stored or ergonomics.

An underside of the inner seal is preferably supported solely by the support surface of the dispensing container.

Advantageously, the inner seal is supported by the support surface in the rest position and the dispensing position.

Preferably the neck has an internal diameter of between 4 and 6 mm and the open top of the dispensing container has a diameter of between 13 and 14 mm.

Preferably the metering valve, with the exception of the valve stem, does not protrude into the neck of the dispensing container. This allows the neck (and if desired the flask portion) of the dispensing container to have a smaller diameter than the metering valve. This helps to reduce the overall size of the dispensing container. In addition, this arrangement helps to reduce the ullage of the valve.

Preferably the biasing means comprises a spring located within the metering chamber volume for biasing the valve stem into the rest position.

Advantageously the valve stem is a one-piece valve stem.

Preferably an inner end of the valve stem is open. Preferably the valve stem further comprises a transfer port through a side wall of the valve stem for allowing flow of fluid into the metering chamber volume from the dispensing container and wherein the inner end of the valve stem comprises a slot separating the valve stem into two legs, the slot extending from the open inner end of the valve stem part-way along the valve stem into communication with the transfer port.

In one embodiment the valve stem comprises only a single transfer port for allowing flow of fluid into the metering chamber volume from the dispensing container.

Preferably the valve stem further comprises one or more external projections which engage an underside of the inner seal on movement of the valve stem from the dispensing position back into the rest position in order to help lift the inner seal back to its rest position in the event of the inner seal undergoing deflection during movement of the valve stem from the rest position into the dispensing position.

Advantageously this helps to maintain the geometry of the inner seal in the rest position so as to help control the metered volume within the chamber. Since the inner seal is supported around its outer periphery by the support surface but is unsupported nearer the inner periphery it will undergo in most cases some inward deflection during movement of the valve stem from the rest position into the dispensing position. Indeed this inward deflection is used during pressure filling of the valve. The external projections help the inner seal to recover to its rest position.

Preferably on assembly of the metering valve with the dispensing container, the inner seal is compressed directly between the support surface of the dispensing container and the metering chamber body.

Preferably an inner end of the metering chamber body comprises an inner recess for receiving the inner seal,
wherein the degree of compression of the inner seal between the support surface and the metering chamber body on assembly of the metering valve with the dispensing container is controllable by altering the depth of the inner recess.

Preferably on assembly of the metering valve with the dispensing container, the outer seal is compressed directly between the ferrule and the metering chamber body.

Preferably an outer end of the metering chamber body comprises an outer recess for receiving the outer seal, wherein the degree of compression of the outer seal between the ferrule and the metering chamber body on assembly of the metering valve with the dispensing container is controllable by altering the depth of the outer recess.

The metering chamber body may further comprise one or more pressure filling channels.

A particular advantage of the present dispensing apparatus is that it may be formed from only seven individual parts, namely: a dispensing container, a one-piece valve stem, a metering chamber body, an outer seal, an inner seal, a spring and a ferrule.

The apparatus may further comprise a dip tub connected to an inner end of the valve stem.

Preferably the metering chamber volume is between 50 and 63 microlitres.

The dispensing apparatus may be a pressurised metered dose inhaler. In particular the dispensing apparatus may be configured as a nasal dispenser, a sub-lingual dispenser, or an inhalatory dispenser. The metering valve may be a capillary retention valve or an open chamber gravity filled valve.

The present invention also provides a metering valve for use in a dispensing apparatus as described above.

A particular advantage of the present metering valve is that it may be formed from only six individual parts, namely: a one-piece valve stem, a metering chamber body, an outer seal, an inner seal, a spring and a ferrule.

The metering valve may be for use in, and the dispensing apparatus may be, a pharmaceutical dispensing device, such as, for example, a pulmonary, nasal, or sub-lingual delivery device. A preferred use of the dispensing apparatus is a pharmaceutical metered dose aerosol inhaler device. The term pharmaceutical as used herein is intended to encompass any pharmaceutical, compound, composition, medicament, agent or product which can be delivered or administered to a human being or animal, for example pharmaceuticals, drugs, biological and medicinal products. Examples include antiallergics, analgesics, antibodies, vaccines, bronchodilators, antihistamines, therapeutic proteins and peptides, antitussives, anginal preparations, antibiotics, anti-inflammatory preparations, hormones, or sulfonamides, such as, for example, a vasoconstrictive amine, an enzyme, an alkaloid, or a steroid, including combinations of two or more thereof. In particular, examples include isoproterenol [alpha-(isopropylaminomethyl) protocatechuyl alcohol], phenylephrine, phenylpropanolamine, glucagon, insulin, DNAse, adrenochrome, trypsin, epinephrine, ephedrine, narcotine, codeine, atropine, heparin, morphine, dihydromorphinone, ergotamine, scopolamine, methapyrilene, cyanocobalamin, terbutaline, rimiterol, salbutamol, flunisolide, colchicine, pirbuterol, beclomethasone, orciprenaline, fentanyl, and diamorphine, streptomycin, penicillin, procaine penicillin, tetracycline, chlorotetracycline and hydroxytetracycline, adrenocorticotropic hormone and adrenocortical hormones, such as cortisone, hydrocortisone, hydrocortisone acetate and prednisolone, insulin, cromolyn sodium, and mometasone, including combinations of two or more thereof.

The pharmaceutical may be used as either the free base or as one or more salts conventional in the art, such as, for example, acetate, benzenesulphonate, benzoate, bicarbonate, bitartrate, bromide, calcium edetate, camsylate, carbonate, chloride, citrate, dihydrochloride, edetate, edisylate, estolate, esylate, fumarate, fluceptate, gluconate, glutamate, glycollylarsanilate, hexylresorcinate, hydrobromide, hydrochloride, hydroxynaphthoate, iodide, isethionate, lactate, lactobionate, malate, maleate, mandelate, mesylate, methylbromide, methylnitrate, methylsulphate, mucate, napsylate, nitrate, pamoate, (embonate), pantothenate, phosphate, diphosphate, polygalacturonate, salicylate, stearate, subacetate, succinate, sulphate, tannate, tartrate, and triethiodide, including combinations of two or more thereof. Cationic salts may also be used, for example the alkali metals, e.g. Na and K, and ammonium salts and salts of amines known in the art to be pharmaceutically acceptable, for example glycine, ethylene diamine, choline, diethanolamine, triethanolamine, octadecylamine, diethylamine, triethylamine, 1-amino-2-propanol-amino-2-(hydroxymethyl)propane-1,3-diol, and 1-(3,4-dihydroxyphenyl)-2 isopropylaminoethanol.

The pharmaceutical will typically be one which is suitable for inhalation and may be provided in any suitable form for this purpose, for example as a solution or powder suspension in a solvent or carrier liquid, for example ethanol, or isopropyl alcohol. Typical propellants are HFA134a, HFA227 and di-methyl ether.

The pharmaceutical may, for example, be one which is suitable for the treatment of asthma. Examples include salbutamol, beclomethasone, salmeterol, fluticasone, formoterol, terbutaline, sodium chromoglycate, budesonide and flunisolide, ipratropium bromide and salbutamol, and physiologically acceptable salts (for example salbutamol sulphate, salmeterol xinafoate, fluticasone propionate, beclomethasone dipropionate, and terbutaline sulphate), solvates and esters, including combinations of two or more thereof. Individual isomers such as, for example, R-salbutamol, may also be used. As will be appreciated, the pharmaceutical may comprise of one or more active ingredients, an example of which is flutiform, and may optionally be provided together with a suitable carrier, for example a liquid carrier. One or more surfactants may be included if desired.

The seals of the metering valve may be formed from any suitable material having acceptable performance characteristics. Preferred examples include nitrile, EPDM and other thermoplastic elastomers, butyl and neoprene.

Other rigid components of the dispensing apparatus, such as the metering chamber body and valve stem may be formed, for example, from polyester, nylon, acetal or similar. Alternative materials for the rigid components of the valve include stainless steel, ceramics, glass and aluminium. The dispensing container may be formed from glass, plastics or a metal such as stainless steel or aluminium.

One or more components of the metering valve may have a layer of one or more polymerised monomers bonded to at least a portion thereof. Preferably the one or more monomers are selected from the group of materials comprising perfluorocyclohexane, perfluoro-hexane, tetrafluoroethylene, trifluoroethylene, vinylidene fluoride, vinylfluoride, fluoroethylene, fluoropropylene, a siloxane, a silazane, and a parylene.

In order that the invention may be fully disclosed, embodiments will now be described, by way of example only, with reference to the accompanying drawings in which:
Figure 1 is a cross-sectional view of a first embodiment of dispensing apparatus according to the present invention in a rest position;
Figure 2 is a cross-sectional view of the dispensing apparatus of Figure 1 in a dispensing position;
Figure 3 is an exploded perspective view of the dispensing apparatus of Figure 1;
Figure 4 is a cross-sectional view of a second embodiment of dispensing apparatus according to the present invention in a rest position;
Figure 5 is a cross-sectional view of a third embodiment of dispensing apparatus according to the present invention in a rest position; and
Figure 6 is a cross-sectional view of a fourth embodiment of metering valve assembly according to the present invention in a rest position.

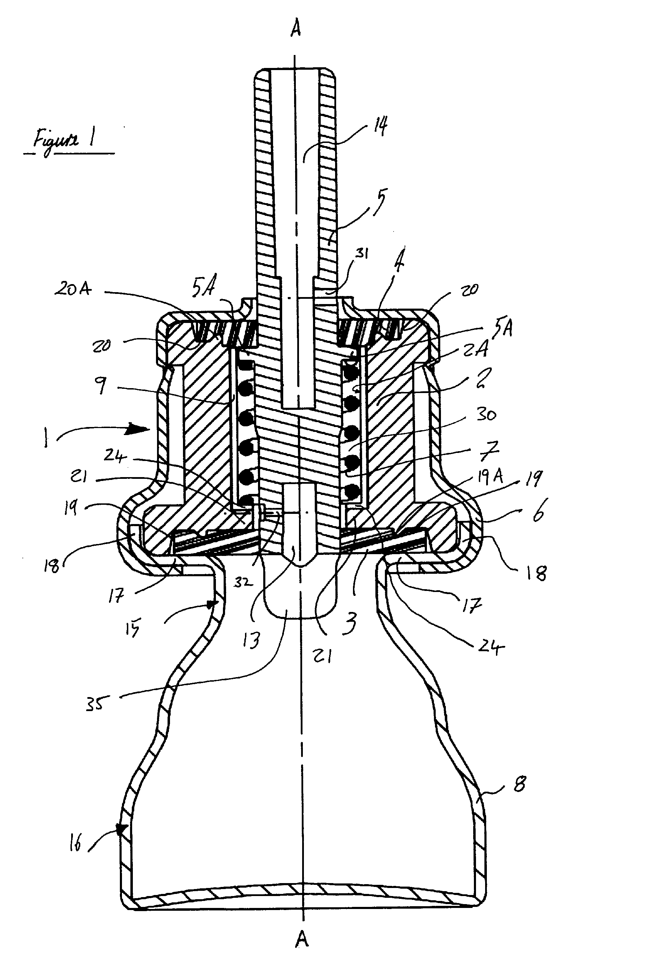

A dispensing apparatus according to the present invention is shown in Figures 1, 2 and 3. The dispensing apparatus generally comprises a metering valve 1 assembled to a dispensing container 8 by means of a ferrule 6. The dispensing container 8 is pressurised by means of a liquefied propellant and contains a product intended for metered dispensation by the metering valve 1.

The metering valve 1 comprises a metering chamber body 2, an inner seal 3, an outer seal 4, a valve stem 5, a spring 7 and a ferrule 6. As it will be understood from the Figures and corresponding description, advantageously the metering valve is formed from only six components and the overall dispensing apparatus is able to be formed from a minimum of seven components (excluding the propellant and product).

The terms inner and outer are used herein to define the position of a feature relative to an inner or an outer end of the metering valve. The inner end of the metering valve is the end closest to the inlet end of the valve stem 5 and the outer end of the metering valve is the end closest to the outlet end of the valve stem 5.

The metering chamber body 2 is cylindrical with a hollow central region which defines a metering chamber volume 30. The valve stem 5 extends through the metering chamber volume 30. An outer end of the metering chamber volume 30 is sealed by the outer seal 4. An inner end of the metering chamber volume is sealed by the inner seal 3. The inner and outer seals are annular and each provide a central aperture through which the valve stem 5 passes in sliding contact.

The metering chamber body 2 includes an inner recess 19 at an inner end of the metering chamber body 2 and an outer recess 20 at the opposed, outer end. The inner recess 19 is provided to receive the inner seal 3 and the outer recess 20 is provided to receive the outer seal 4. The recesses 19, 20 are sized to control the overall degree of compression of the inner and outer seals 3,4 as described further below. The inner and outer recesses 19, 20 are also each provided with a raised bead 19A, 20A which bites into the respective seal on assembly to help control the degree of localised compression of the seals.

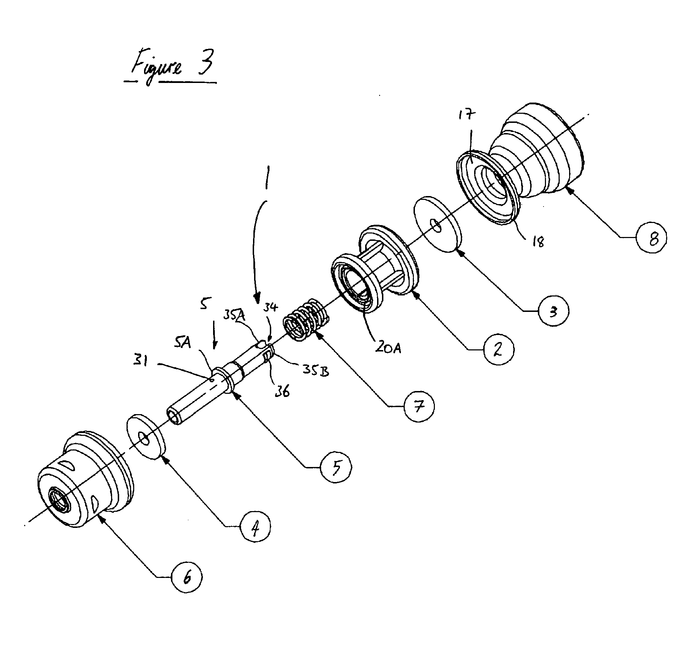

The valve stem 5 is a unitary elongated member and includes two separate bores, 13, 14 and an external flange 5A positioned part way along the length of the stem. The inner bore 13 is provided with an inlet port 32 in a side wall. The inlet port 32 provides an inlet to the metering chamber volume 30 at a first, rest, position of the valve stem 5 with respect to the metering chamber volume 30. The outer bore 14 is provided with an outlet port 31 in a side wall. The outlet port 31 provides an outlet from the metering chamber volume 30 at a second, dispensing, position of the valve stem 5 with respect to the metering chamber volume 30. Accordingly, the valve stem 5 is moveable between rest and dispensing positions of the metering chamber volume 30, to provide dispensation of a product through the metering valve 1. As shown in Figure 3 an inner end of the valve stem 5 is provided with an open end formed by a slot 34 which separates the valve stem into two legs 35A and 35B. An external projection 36 is provided on an external face of each leg 35A and 35B. The external projection 36 provides an upwardly facing ledge, the use of which will be described below.

The spring 7 is provided to bias the valve stem 5 into the rest position of Figure 1. The spring 7 is located entirely within the metering chamber volume 30 and extends from an underside of the flange 5A to an inner end of the metering chamber body 2 where an inwardly directed flange 21 is provided which forms an abutment surface for the spring 7.

In the embodiment shown in Figure 1 the spring 7 contacts directly the flange 5A and the flange 21. However, in an alternative, as long as the spring 7 biases the flanges 5A, 21 apart, there is no specific requirement that the spring 7 contact those flanges 5A, 21 directly.

A radially inner surface 2A of the metering chamber body 2 is provided with a number of flutes 9 or recessed channels. As a result the radially inner surface 2A comprises an undulating surface with a varying internal diameter. Thus channels are formed through which fluid may pass unimpeded by the spring. Further, a number of flutes 24 or recessed channels are provided on an upper surface of the flange 21, again, to provide a channel through which fluid may pass. Preferably the flutes 24 are a continuation of the flutes 9. In particular, for when the spring 7 is fully-compressed (coil-bound) the channels allow fluid to flow around the outside of the spring 7 from within the metering chamber volume 30 to reach the outlet port 31 of the valve stem 5. This is particularly advantageous when pressure filling the dispensing apparatus as described further below.

The dispensing container 8 comprises a flask portion 16 having a sealed lower end, a relatively narrow neck 15 and an open top. The flask portion 16 defines a storage volume. The neck 15 is provided with a reduced-diameter relative to the flask portion 16 and the open top. The open top comprises an out-turned flange 17 which extends radially outwardly from an upper end of the neck 15 and terminates in an up-turned rim 18. The out-turned flange 17 defines a support surface for the metering valve as described below. In particular, the out-turned flange 17 is perpendicular to the longitudinal axis of the container (and also of the metering valve 1 when assembled therewith), shown in Figure 1 by the line A-A. The out-turned flange 17 is a substantially flat annulus. Thus, the container 8 comprises a relatively small internal diameter opening within the neck 15 and a relatively large internal diameter opening defined by the up-turned rim 18.

To assembly the dispensing apparatus the metering valve 1 is first assembled by inserting the spring 7 and valve stem 5 into the metering chamber body 2 and locating the inner seal and outer seal into their respective recesses. The ferrule 6 is located over the metering valve 1. The metering valve is then located in the open top of the dispensing container 8 as shown in Figure 1. The inner end of the metering chamber body 2 and the inner seal 3 contact and are supported by the support surface of the out-turned flange 17. The metering chamber body 2 does not extend down into the neck 15 of the container 8 (although the inner end of the valve stem 5 does protrude slightly into the neck 15).

The ferrule is then crimped to the container 8 to fix the metering valve in place. In particular, and as can be seen in the Figures, the ferrule 6 is crimped under the external shoulder formed by the out-turned flange 17 of the open mouth. On crimping the ferrule 6, the inner seal 3 and outer seal 4 are compressed. The outer seal 4 is compressed between the ferrule 6 and the outer recess 20 of the metering chamber body 2. The inner seal 3 is compressed by direct contact between the support surface of the out-turned flange 17 and the inner recess 19 of the metering chamber body 2. The overall degree of compression of the seals 3, 4 can be controlled by adjusting the depth of the recesses 19, 20 as the seals will generally be compressed to the point where the ferrule contacts the rigid material of the metering chamber body 2 at the outer end as shown in Figure 1. The inner and outer seals 3, 4 seal respective inner and outer ends of the metering chamber volume 30. In addition, the inner seal 3 acts as a gasket seal between the metering valve 1 and the dispensing container 8 preventing leakage to atmosphere of product from the storage volume of the container 8 between the metering valve and the ferrule 6.

The inner seal 3 is supported around its outer periphery by the out-turned flange 17 but is free to deflect inwardly under movement of the valve stem 5. An underside of the inner seal 3 is located just above or in contact with the ledges of the external projections 36 on the valve stem 5.

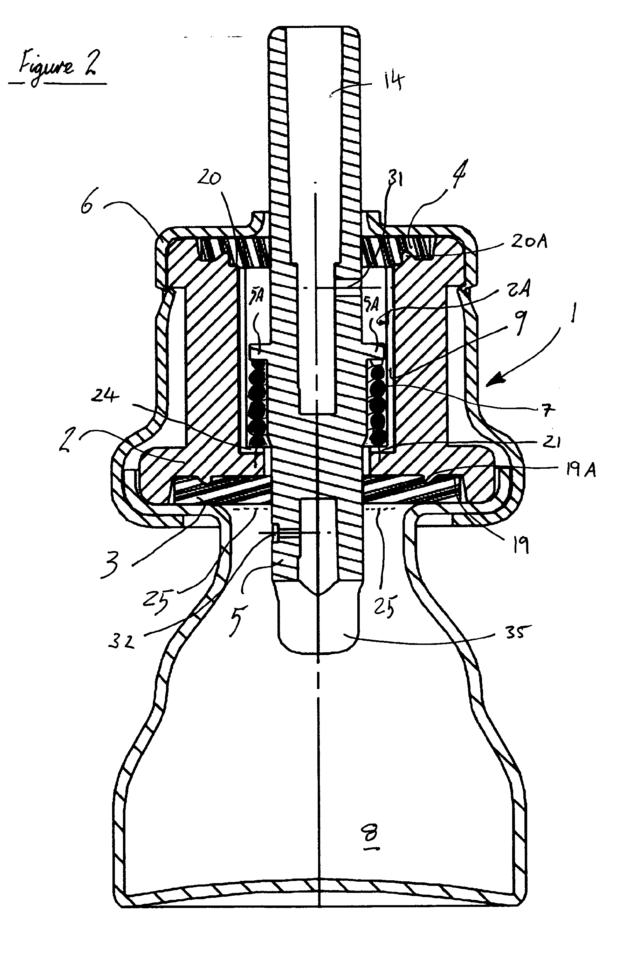

The dispensing container can be pressure filled by overcoming the seal between the valve stem 5 and the inner seal 3. First the valve stem 5 is depressed into the dispensing position of Figure 2. Next product is injected under pressure into the outlet bore 14 of the valve stem 5. The product passes through the outlet port 31, into the metering chamber volume 30. The fluid progresses to the inner end of the metering chamber volume 30 (via the flutes 9 and 24 if necessary). The pressure applied by the fluid causes the inlet seal 3 to deflect inwardly (as shown by the dotted lines 25 in Figure 2) breaking the sealing contact between the inner seal 3 and the valve stem 5 allowing the fluid to pass into the storage volume of the flask portion 16 of the dispensing container 8.

In use metered doses of product are dispensed by moving the valve stem from the rest position of Figure 1 into the dispensing position of Figure 2. Typically the inward movement of the valve stem 5 can drag on the inner seal causing it to deflect downwardly slightly. Advantageously, the ledges of the external projections 36 on the valve stem 5 contact the inner seal 3 on the return stroke of the valve stem 5 thereby lifting the inner seal 3 back into its rest position.

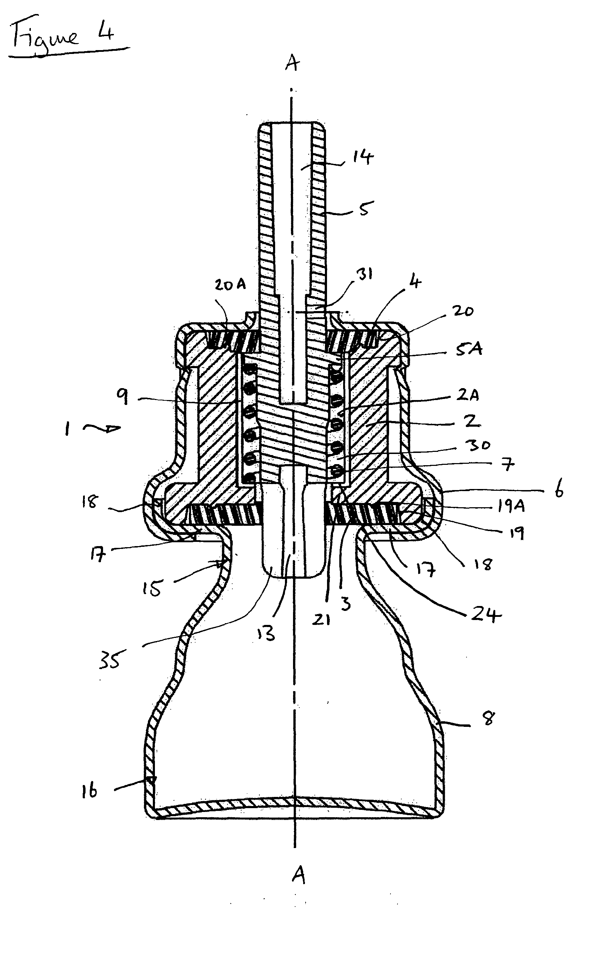

The metering valve 1 may be designed as a capillary retention valve (as shown in Figures 1 to 3) where the metering chamber volume 30 is filled immediately after dispensation of a prior dose and retains its fluid until a subsequent actuation. Alternatively the valve may be designed as a fast-filling valve as shown in the second embodiment of Figure 4 where the metering chamber volume 30 is substantially empty when the apparatus is orientated with the valve stem 5 uppermost and refills quickly on inversion of the apparatus immediately prior to actuation. Like numbering has been used for the first and second embodiments. As above the inner end 35 of the valve stem has a slot 34 which separates the valve stem into two legs 35A and 35B. However, the slot 34 is longer and thinner and allows free communicate with the metering chamber volume in the rest position. The metering chamber volume is sealed on depression of the valve stem when the enlarged diameter of the mid-portion of the valve stem contacts the inner seal 3.

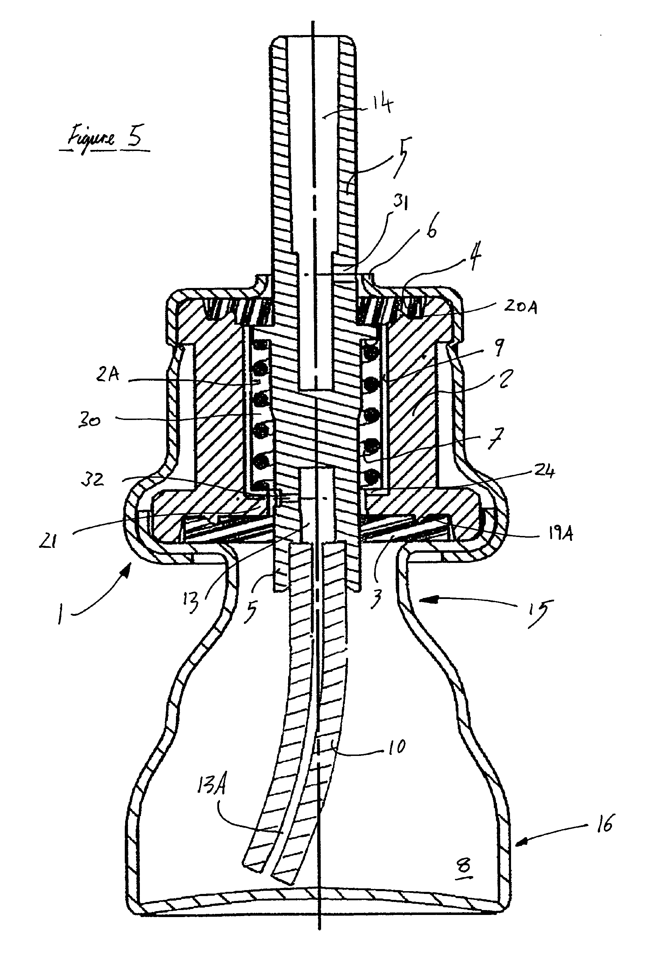

Figure 5 shows a third embodiment of dispensing apparatus according to the present invention. Like references have been used to identify common features, which common features will not be discussed in detail. In particular, the differences between the embodiment shown in Figure 5 and the first embodiment of the invention - shown in Figures 1 to 3 - will be described.

A dip-tube 10 is provided and is connected to an inner end of the valve stem 5. As such, the inner bore 13 now comprises the bore 13a, provided through the dip-tube 10, which leads on to the inner bore 13 of the valve stem 5. During operation of the metering valve 1, the dip-tube 10 is moveable within the container 8, in register with movement of the valve stem 5. Preferably, the dip-tube 10 is a micro-bore dip-tube which prevents back-flow of fluid down the dip-tube between actuations of the metering valve.

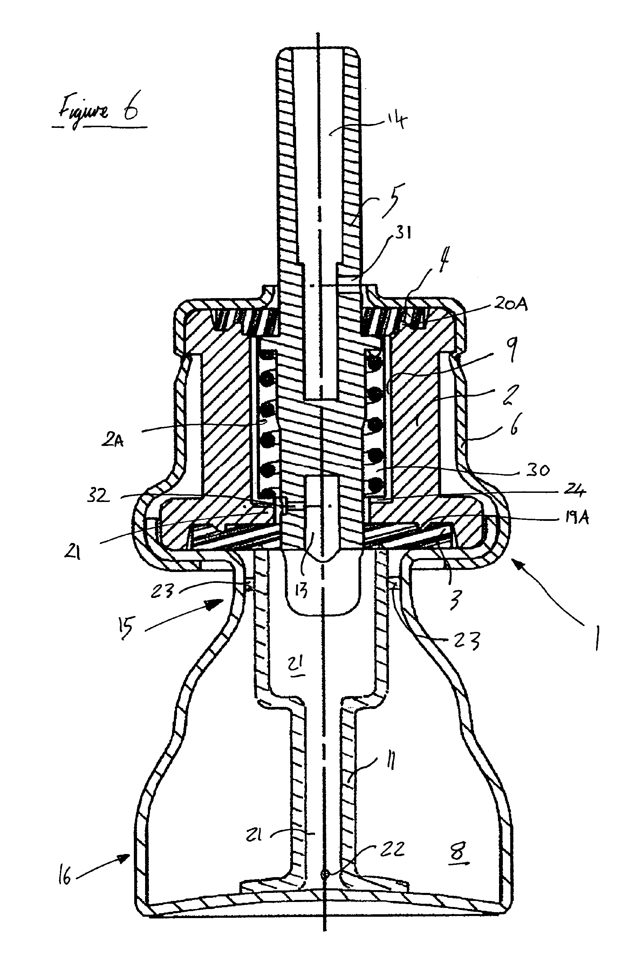

Figure 6 shows a fourth embodiment of dispensing apparatus according to the present invention. Like references have been used to identify common features, which common features will not be discussed in detail. In particular, the differences between the embodiment shown in Figure 6 and the first three embodiments will be described.

In the embodiment shown in Figure 6, a dip-tube 11 is provided. Unlike the dip-tube 10 shown in Figure 5, the dip-tube 11 is stationary and is not connected directly to the valve stem 5. As such, the dip-tube 11 is fixed within the container 8, at a bottom of the container 8 and is sealed against the inner seal 3 at or around the neck 15 of the container 8. An internal bore 21 of the dip-tube 11 and an aperture 22 provide a flow path for fluid to enter the bore 13 of the valve stem 5. For stability, a spacer 23 is provided around the dip-tube 11 to prevent movement of the dip-tube 11 in the region of an internal surface of the neck 15. The embodiment provides a permanent vacuum on fluid in the container 8, preventing backflow of fluid.

The provision of the dip-tubes in the embodiments shown in Figures 5 and 6 allows the apparatus to be used in the orientation shown in those Figures, i.e. in a non-inverted state. Accordingly, these embodiments may be particularly beneficial in nasal applicators. The embodiments shown in Figures 1 to 4 find particular use in aerosol dispensers, including medical inhalers.

## Claims

1. A dispensing apparatus comprising a dispensing container assembled with a metering valve;
the dispensing container defining a storage volume for a product, the dispensing container comprising a neck terminating in an open top for receiving the metering valve; the open top extending radially outwardly relative to the neck to define a support surface;
the metering valve comprising a valve assembly and a ferrule wherein the valve assembly comprises:
a metering chamber body surrounding a metering chamber volume;
an inner seal, for sealing an inlet of the metering chamber volume;
an outer seal, for sealing an outlet of the metering chamber volume;
a valve stem; and
a biasing means for biasing the valve stem;
the valve stem being movable between a dispensing position, in which the outlet of the metering chamber volume is open and the inlet of the metering chamber volume is closed, and a rest position, in which the outlet of the metering chamber volume is closed and the inlet of the metering chamber volume is open;
wherein the inner seal is supported by the support surface of the open top of the dispensing container when the metering valve is assembled with the dispensing container.

2. Dispensing apparatus as claimed in claim 1 wherein direct contact between the inner seal and the dispensing container acts as a gasket seal between the dispensing container and the metering valve.

3. Dispensing apparatus as claimed in claim 1 or claim 2 wherein the support surface is formed by a portion of the open top of the dispensing container located between the neck and a distal rim of the open top.

4. Dispensing apparatus as claimed in any preceding claim wherein the open top defines a recess for locating the metering valve in engagement with the dispensing container.

5. Dispensing apparatus as claimed in claim 4 wherein the recess is bounded by an upturned rim of the open top of the dispensing container.

6. Dispensing apparatus as claimed in claim 5 wherein the support surface is formed by a portion of the open top of the dispensing container located between the neck and the upturned rim of the open top.

7. Dispensing apparatus as claimed in any preceding claim wherein the dispensing container further comprises a flask portion, wherein the minimum diameter of the neck is smaller than the maximum diameter of the flask portion.

8. Dispensing apparatus as claimed in any preceding claim wherein an underside of the inner seal is supported solely by the support surface of the dispensing container.

9. Dispensing apparatus as claimed in any preceding claim wherein the inner seal is supported by the support surface in the rest position and the dispensing position.

10. Dispensing apparatus as claimed in any preceding claim wherein the metering valve, with the exception of the valve stem, does not protrude into the neck of the dispensing container.

11. Dispensing apparatus as claimed in any preceding claim wherein the valve stem further comprises one or more external projections which engage an underside of the inner seal on movement of the valve stem from the dispensing position back into the rest position in order to help lift the inner seal back to its rest position in the event of the inner seal undergoing deflection during movement of the valve stem from the rest position into the dispensing position.

12. Dispensing apparatus as claimed in any preceding claim wherein on assembly of the metering valve with the dispensing container, the inner seal is compressed directly between the support surface of the dispensing container and the metering chamber body.

13. Dispensing apparatus as claimed in claim 12 wherein an inner end of the metering chamber body comprises an inner recess for receiving the inner seal, wherein the degree of compression of the inner seal between the support surface and the metering chamber body on assembly of the metering valve with the dispensing container is controllable by altering the depth of the inner recess.

14. Dispensing apparatus as claimed in any preceding claim comprising only seven individual parts, namely: a dispensing container, a one-piece valve stem, a metering chamber body, an outer seal, an inner seal, a spring and a ferrule.

15. A metering valve for use in a dispensing apparatus as claimed in any preceding claim.
